Europäisches Patentamt

European Patent Office

Office européen des brevets

(11) Publication number: **0 665 022 A1**

# EUROPEAN PATENT APPLICATION

(21) Application number: 94300663.5

(22) Date of filing: 28.01.94

(51) Int. Cl.⁶: **A61L 25/00**, A61L 31/00, C08L 5/08, C08B 37/00

(43) Date of publication of application:
02.08.95 Bulletin 95/31

(84) Designated Contracting States:
**CH DE ES FR GB IT LI SE**

(71) Applicant: **PRODEX, INC.**
**3490 U.S. 1**
**Princeton,**
**New Jersey 08540 (US)**

(72) Inventor: **Nichols, Joseph**
**Princeton**
**New Jersey (US)**

(74) Representative: **Seaborn, George Stephen**
**c/o Edward Evans & Co.**
**Chancery House**
**53-64 Chancery Lane**
**London WC2A 1SD (GB)**

(54) Viscoelastic solution of N,O-carboxymethyl chitosan for ophthalmic use.

(57) Viscoelastic solutions of N,O-carboxymethyl chitosan and its salts are disclosed. The viscoelastic solution of N,O-carboxymethyl chitosan are used in a variety of medical applications, particularly ophthalmic surgery.

EP 0 665 022 A1

BACKGROUND OF THE INVENTION

N,O-Carboxymethyl chitosan

This invention relates to N,O-carboxymethyl chitosan and, more specifically, this invention relates to a viscoelastic solution of N,O-carboxymethyl chitosan and its salts, and its use in a variety of medical applications, particularly ophthalmic surgery.

N,O-carboxymethyl chitosan, *per se*, is known. N,O-carboxymethyl chitosan and its preparation are described by Ernest R. Hayes in U.S. Patent No. 4,619,995, assigned to Nova Chem Limited, which is incorporated herein in its entirety by reference. The primary use taught by U.S. Patent No. 4,619,995 for N,O-carboxymethyl chitosan is as a selectively permeable film or membrane. It is also taught that N,O-carboxymethyl chitosan will sequester ions of various heavy metal elements at low concentrations of the heavy metal ions. At higher concentrations of the heavy metal ions, a stiff gel forms, thus suggesting that aqueous solutions of N,O-carboxymethyl chitosan may be used to plug porous underground strata. N,O-carboxymethyl chitosan is also known to cross-link when reacted with glyoxal and other dialdehydes. These properties were also elaborated by Davies, Elson, and Hayes, "N,O-Carboxymethyl Chitosan, a New Water Soluble Chitin Derivative," presented at the 4th International Conference on Chitin and Chitosan, 22-24 August, 1988, Trondheim, Norway.

Ophthalmic Uses of Viscoelastic Agents

Viscosurgical techniques are employed in performing delicate eye surgery. Viscoelastic agents were initially used for vitreous replacement. Their application to cataract surgery revolutionized ophthalmic surgery. They facilitate intraocular manipulation and space maintenance. They protect the corneal endothelium, maintain the anterior chamber, separate tissue surfaces and allow for manipulation of the iris.

The viscoelastic agent which is most widely used in ophthalmic surgery today is hyaluronic acid and its salts, although other materials such as chondroitin sulfate, hydroxypropyl methylcellulose, polyacrylamide. See, Richard L. Lindstrom, Rhondi Larson & Debra Skelnik, "Viscoelastic Agents," New Ophthalmic Drugs, Ophthalmology Clinics of North America, 2, 1, 173-186 (March 1989). Chemically modified collagen is caught for this purpose in U.S. Patent No. 4,713,446, to Dale P. DeVore, and assigned to 3M.

According to T. J. Liesegang, M.D., "Viscoelastic Substances in Ophthalmology," Survey of Opthalmology, 34, 4, 268-293 (Jan. - Feb., 1990), the specific "chemical requirements [of viscoelastic substances for use in ophthalmology] are that the viscoelastic substance be inert, electrolyte balanced, at the same osmolality and colloid osmotic pressure as the cornea or aqueous fluid, pH buffered, soluble in water, highly purified, free of particles and transparent for use within the anterior chamber. It should be easy to instill and to remove, and should be removable from the eye biologically."

SUMMARY OF THE INVENTION

It has been discovered that N,O-carboxymethyl chitosan and its derivatives meet the "requirements" laid dawn by Dr. Liesegang. The present invention, therefore, provides an aqueous viscoelastic solution of N,O-carboxymethyl chitosan. It is especially important for ophthalmic purposes that the viscoelastic solution of N,O-carboxymethyl chitosan be sterile and non-pyrogenic. The present invention is also concerned with the use of the viscoelastic solution of N,O-carboxymethyl chitosan for various medical purposes, most importantly for ophthalmic surgery. More particularly, a method of replacing at least a portion of the aqueous or vitreous humor in a human or animal eye comprising introducing into the aqueous or vitreous chamber of the human or animal eye an effective amount of the viscoelastic N,O-carboxymethyl chitosan solution. Also, a method of inserting an intraocular lens into the anterior or posterior chamber of a human or animal eye comprising introducing into the anterior or posterior chamber an amount of the viscoelastic N,O-carboxymethyl chitosan solution sufficient to reduce the trauma which normally results from inserting an intraocular lens into the chamber without the use of a viscoelastic ophthalmic aid.

In other medical applications, the present invention involves a method of preventing fibrous tissue formation and consequent development of adhesion and scars which may occur in healing tissue during the normal healing process comprising introducing into a surgical site, either during surgery, or post-operatively an effective amount of a viscoelastic N,O-carboxymethyl chitosan solution. Similarly, a method of reducing inflammation and promoting the normal healing of soft tissues and cartilage comprising introducing by injection into the affected soft tissue or cartilage an effective amount of a viscoelastic N,O-carboxymethyl chitosan solution, and a method of enhancing normal joint and tendon function in a human or animal by lubricating the joint or tendon comprising introducing into the synovial space associated with the joint or tendon an effective lubricating amount of a viscoelastic N,O-carboxymethyl chitosan solution.

DETAILED DESCRIPTION OF THE INVENTION

Preparation at Viscoelastic N,O-Carboxymethyl Chitosan Solutions

For the purposes of this invention, N,O-carboxymethyl chitosan, its physiologically acceptable salts, and cross-linked N,O-carboxymethyl chitosan may be used Exemplary of such salts are the acetate, lactate, citrate, and pyroglutamate salts. The N,O-carboxymethyl chitosan may be cross-linked with dialdehydes such as glyoxal, malondialdehyde, phthalic dicarboxaldehyde, gluteraldehyde, and the like. The N,O-carboxymethyl chitosan or cross-linked N,O-carboxymethyl chitosan is dissolved in a suitable vehicle, such as isotonic saline or Balanced Salt Solution (BSS) at a concentration of between about 0.5 and about 3% by weight, preferably about 1 to about 3% of the N,O-carboxymethyl chitosan and about 0.5 to about 2% of the cross-linked material. Salts of N,O-carboxymethyl chitosan would be dissolved in glycerin at a concentration of between about 0.2 and about 3% by weight, preferably about 0.5 to about 2%.

The eye is a very sensitive organ and traces of foreign material or non-sterile or pyrogenic solutions have a negative effect on inflammatory reaction in the aqueous or vitreous. Thus, it is important that, for ophthalmic use, the viscoelastic solution of N,O-carboxymethyl chitosan be sterile and non-pyrogenic. Sterilization is done by membrane filtration or flash autoclaving in closed containers. In membrane filtration, the material is passed through 0.2 or 0.45 micron filters. The samples used in the following examples were sterilized using Millipore Co. membranes in steel containers. The heated N,O-carboxymethyl chitosan solution was filtered through heated equipment under 100 lbs./in.$^2$ (6.8 atm.) nitrogen gas pressure.

The acid salts of N,O-carboxymethyl chitosan such as the acetate, the lactate, or the pyroglutamate give much higher viscosities in solution than the N,O-carboxymethyl chitosan itself. They give gels at a relatively low concentration. However the acid salts will not dissolve in salt solutions such as BSS or isotonic saline. To obtain the required osmolality for use in the eye, a glycerin solution can he used. At 2 to 2.2% glycerin in water, the osmolality is close to 340. The acid salts off N,O-carboxymethyl are not lubricious as is N,O-carboxymethyl chitosan in solution.

The ideal pH for solutions to be used in the eye is between about 7.2 and about 7.4. The osmolality which is ideal to prevent corneal swelling is between about 300 and about 340.

The N,O-carboxymethyl chitosan used in the following examples was protein free as proven by amino acid analysis. No amino acids were present in samples submitted for analysis. This is important since the presence of proteins would give rise to antigenicity. The N,O-carboxymethyl chitosan polysaccharide itself is non-antigenic. The results of tests run on samples which proved to be impure and pyrogenic were discarded.

Example 1

Samples of viscoelastic N,O-carboxymethyl chitosan solutions were made for testing as follows:

Sample No. 200-242B. N,O-Carboxymethyl chitosan Lot No. S1349 from Nova Chem Ltd. (Brookfield viscosity of a 1% solution in water - 925 cps.) was dissolved in BSS at a concentration of 2.5%. The solution was filtered through a Millipore Polygard membrane and then through a 5μ and a 0.45μ Durapore (Millipore Co.) membrane successively. The pH was 7.88 and the osmolality was 339.

Sample No. 300-37B. N,O-Carboxymethyl chitosan Lot No. S1472 from Nova Chem Ltd. (Brookfield viscosity of a 1% solution in water - 1750 cps.) was dissolved in 90% BSS-10% distilled water at a concentration or 2%. The solution was filtered through a Millipore Polygard membrane and than through 5μ and 0.45μ membranes successively. The pH was 7.78 and the osmolality was 399.

Sample No. 200-302. N,O-Carboxymethyl chitosan Lot No. S1406 from Nova Chem Ltd. (Brookfield viscosity of a 1% solution in water - 1850 cps.) was dissolved in 85% BSS-15% distilled water at a concentration of 1.5% The solution was filtered through a Millipore Polygard membrane and then through 5μ and 0.45μ membranes successively. The pH was 7.3 and the osmolality was 301.

Sample No. 300-39A. N,O-Carboxymethyl chitosan acetate Lot No. C659 from Nova Chem Ltd. (viscosity >120,000 cps.) was dissolved in a phosphate buffered 2.2% solution of glycerin at a concentration of 1%. The solution was filtered through a Millipore Polygard membrane and then through 5μ and 0.45μ Durapore membranes successively. The pH was 7.11 and the osmolality was 306.

Testing of Viscoelastic N,O-Carboxymethyl Chicosan Solutions

Example 2

A study was undertaken to evaluate ocular inflammation produced by sample 200-242B against a control of Healon® (sodium hyaluronate from Pharmacia, lot No. PK66220) after anterior chamber injection in New Zealand white rabbits. The parameters studied were corneal thickness, intraocular pressure and ocular irritation and inflammation as graded by the McDonald-Shadduck score system. Animals wore examined prior to and at 0, 1, 3, 5, 24, and 48 hours after injection of the test material into the anterior chamber. At each time interval the treated eyes were examined and scored for ocular reactions according to the modified McDonald-Shadduck score system. This study was performed to provide information on the inflammatory response to the injected materials in the rabbit model. The study was conducted following FDA Good laboratory Practices (part 58 of 21 CFR).

Adult rabbits (5-6 lbs.) were used for this aqueous replacement study. One day prior to the study, by slit lamp evaluation both eyes of each animal were examined using fluorescein dye to determine the condition of the corneal epithelium. The corneal epithelium was evaluated to determine if any corneal abrasion, ulceration or irregularities were present. On the day of the study, each animal was again evaluated prior to the injection of the test material. Fluorescein dye was not used in this pre-injection evaluation. Animals exhibiting pre-existing corneal, lens or conjunctival injury, irritation or irregularity were not used in this study.

General anesthesia was achieved by intramuscular infection of Rompun (10 mg/kg body weight) and Ketamine HCl (50 mg/kg body weight). The pupil was dilated with .05 ml of 1% Cyclopentolate HCl and 2.5% Phenylephrine HCl administered topically. One drop of Proparacaine .5% was administered topically prior to aqueous injection. All injected solutions were sterile and administered undiluted. Healon was purchased from Pharmacia. All samples were stored at 4-6°C until the time of use.

The eye was prepared for surgery and a 25-gauge butterfly infusion needle was inserted at the 10 o'clock position, 0.2 mm anterior to the limbus. To prevent collapse of the anterior chamber, the outflow tubing remained capped until the injection of the test material was initiated. A 1 cc syringe with a 27-gauge needle was inserted at the 2 o'clock position, 0.2 mm anterior to the limbus. The beveled edges of the needles were positioned away from the corneal endothelium. The anterior chamber was filled with 0.150 cc of the test material. After replacement of the aqueous humor with the test material, the outflow and the inflow needles were removed. The intraocular pressure was monitored with a pneumotonometer. Corneal thickness was measured with an ultrasound pachymeter and inflammation and irritation was scored utilizing the McDonald-Shadduck evaluation system. Eyes were evaluated prior to injection, 0, 1, 3, 5, 24 and 48 hours post-injection.

The modified McDonald-Shadduck score system is as follows:

**Conjunctival Congestion**

0 = Normal. May appear blanched to reddish pink without perilimbal injection (except at 12:00 and 6:00 o'clock positions) with vessels of the palpebral and bulbar conjunctival easily observed.

1+ = A flushed, reddish color predominantly confined to the palpebral conjunctiva with some perilimbal injection but primarily confined to the lower and upper parts of the eye from the 4:00 to 7:00 and 11:00 to 1:00 o'clock positions.

2+ = Bright red color of the palpebral conjunctiva with accompanying perilimbal injection covering at least 75: of the circumference of the perilimbal region.

3+ = Dark, beefy red color with congestion of both the bulbar and the palpebral conjunctiva along with pronounced perilimbal injection and the presence of petechia on the conjunctiva. The petechia generally predominate along the nictitating membrane.

**Conjunctival Swelling**

0 = Normal or no swelling of the conjunctival tissue.

1+ = Swelling above normal without eversion of the lids (can be easily ascertained by noting that the upper and lower eyelids are positioned as in the normal eye): swelling generally starts in the lower cul-de-sac near the inner canthus, which needs slit-lamp examination.

2+ = Swelling with misalignment of the normal approximation of the lower and upper eyelids; primarily confined to the upper eyelid so that in the initial stages the misapproximation of the eyelids begins by partial eversion of the upper eyelid. In this stage, swelling is confined generally to the eyelid, although it exists in the lower cul-de-sac.

3+ = Swelling definite with partial eversion of the upper and lower eyelids essentially equivalent. This can be easily ascertained by looking at the animal head-on and noticing the position of the eyelids; if the eye margins do not meet, eversion has occurred.

4 + = Eversion of the upper eyelid is pronounced with less pronounced eversion of the lower eyelid. It is difficult to retract the lids and observe the perilimbal region.

**Conjunctival Discharge**

Discharge is defined as a whitish, grey precipitate, which should not be confused with the small amount of clear, mucoid material that can be formed in the medial canthus of a substantial number of rabbit eyes. This material can be removed with a cotton swab before the animals are used.

0 = Normal. No Discharge.

1 + = Discharge above normal and present on the inner portion of the eye but not on the lids or hairs of the eyelids. One can ignore the small amount that is in the inner and outer canthus if it has not been removed prior to starting the study.

2 + = Discharge is abundant, easily observed, and has collected on the lids and around the hairs of the eyelids.

3 + = Discharge has been flowing over the eyelids so as to wet the hairs substantially on the skin around the eye.

**Aqueous Flare**

The intensity of the Tyndall phenomenon is scored by comparing the normal Tyndell effect observed when the slit-lamp beam passes through the lens with that seen in the anterior chamber. The presence of aqueous flare is presumptive evidence of breakdown of the blood-aqueous barrier.

0 = The absence of a visible light bean in the anterior chamber (no Tyndall effect).

1 + = The Tyndall effect is barely discernible. The intensity of the light beam in the anterior chamber is less than the intensity of the slit beam as it passes through the lens.

2 + = The Tyndall beam in the anterior chamber is easily discernible and is equal in intensity to the slit-beam as it passes through the lens.

3 + = The Tyndall beam in the anterior chamber is easily discernable; its intensity is greater than the intensity if the slit beam is passed through the lens.

**Iris Involvement**

In the following definitions the primary, secondary, and tertiary vessels are utilized as an aid to determining a subjective ocular score for iris involvement. The assumption is made that the greater the hyperemia of the vessels and the more the secondary and tertiary vessels are involved, the greater the intensity of iris involvement.

0 + Normal without any hyperemia of the iris vessels. Occasionally around the 12:00 to 1:00 o'clock position near the pupillary border and the 6:00 and 7:00 o'clock position near the pupillary border there is a small area around 1-3 mm in diameter in which both the secondary and tertiary vessels are slightly hyperemic.

1 + = Minimal injection of secondary vessels but not tertiary. Generally, it is uniform, but may be of greater intensity at the 1:00 or 6:00 o'clock position. If it is confined to the 1:00 or 6:00 o'clock position, the tertiary vessels must be substantially hyperemic.

2 + = Minimal injection of the tertiary vessels and minimal to moderate injection of secondary vessels.

3 + = Moderate injection of the secondary and tertiary vessels with slight swelling of the iris stroma. This gives the iris surface a slightly rugose appearance, which is usually most prominent near the 3:00 and 9:00 o'clock positions.

4 + = Marked injection of the secondary and tertiary vessels with marked swelling of the iris stroma. The iris appears rugose; may be accompanied by hemorrhage (hyphema) in the anterior chamber.

**Cornea**

The scoring scheme measures the severity of the corneal cloudiness and the area of the cornea involved. Severity of the corneal cloudiness is graded as follows:

0 = Normal cornea. Appears with the slit-lamp as having a bright gray line on the epithelial surface and a bright gray line in the endothelial surface with a marblelike gray appearance of the stroma.

1 + = Some loss of transparency. Only the anterior half of the stroma is involved as observed with an optical section of the slit-lamp. The underlying structures are clearly visible with diffuse illumination, although some cloudiness can be readily apparent with apparent with diffuse illumination.

2 + = Moderate loss of transparency. In addition to involving the anterior stroma, the cloudiness extends all the way to the endothelium. The stroma has lost its marblelike appearance and is homogeneously white. With diffuse illumination the underlying structures are clearly visible.

3 + = Involvement of the entire thickness of the stroma. with optical section, the endothelial surface is still visible. However, with diffuse illumination the underlying structures are just barely visible, to the extent that the observer is still able to grade flare, iritis, observe pupillary response, and note lenticular changes.

4 + = Involvement of the entire thickness of the stroma. With the optical section, one cannot clearly visualize the endothelium. With diffuse illumination, the underlying structures cannot be seen. Cloundiness removes the capacity for judging and grading aqueous flare, iritis, lenticular changes, and pupillary response. The surface area of the cornea relative to the area of cloudiness is divided into 5 grades from 0 to 4 + .

0 = Normal cornea with no area of cloudiness.

1 + = 1% - 25% area of stromal cloudiness.

2 + = 26% - 50% area of stromal cloudiness.

3 + = 51% - 75% area of stromal cloudiness.

4 + = 76% - 100% area of stromal cloudiness.

**Pannus**

Pannus is vascularization or the penetration of new blood vessels

into the corneal stroma. The vessels are derived from the limbal vascular loops.

0 = No pannus.

1 + = Vascularization is present but vessels have not invaded the entire corneal circumference. Where localized vessel invasion has occurred, they have not penetrated beyond 2 mm.

2 + = Vessels have invaded 2 mm or more bound the entire corneal circumference.

**Fluorescein Staining**

The use of fluorescein is a valuable aid in defining epithelial damage. The area can be judged on a 0 to 4 + scale using the same terminology as for corneal cloudiness.

0 = Absence of fluorescein staining.

1 + = Slight fluorescein staining to a small focus. With diffuse illumination the underlying structures are easily visible. The outline of the pupillary margin is as if there were not fluorescein staining.

2 + = Moderate fluorescein staining confined to a small focus. With diffuse illumination the underlying structures are clearly visible, although there is some loss of detail.

3 + = Marked fluorescein staining. Staining may involve a larger portion of the cornea with diffuse illumination underlying structures are barely visible but are not completely obliterated.

4 + = Extreme fluorescein staining. With diffuse illumination the underlying structures cannot be observed.

Table 1

| Intraocular Pressure Evaluation | | | | | | |
|---|---|---|---|---|---|---|
| | Pre | 1 Hour | 3 Hours | 5 Hours | 24 Hours | 48 Hours |
| | mm Hg | mm Hg | mm Hg | mm Hg | mm Hg | mm Hg |
| 200-242B | 15.8 12.9 | 5.9 37.6 | 7.1 25.6 | 5 25.3 | 9.5 14.2 | 25.7 24.7 |
| Mean ± SD | 14.4 ±2.1 | 21.8 ±22.4 | 16.4 ±13.1 | 15.2 ±14.4 | 11.9 ±3.3 | 25.2 ±.7 |
| Healon PK66220 | 11.6 15.6 | 11.6 12.4 | 6 11.1 | 9.5 13 | 10.6 8.5 | 14.3 10.9 |
| Mean ± SD | 13.6 ±2.8 | 12.0 ±0.6 | 8.6 ±3.6 | 11.3 ±2.5 | .69 ±1.5 | 12.6 ±2.4 |

Table 2

| Corneal Thickness Evaluation | | | | | | |
|---|---|---|---|---|---|---|
| | Pre | 1 Hour | 3 Hours | 5 Hours | 24 Hours | 48 Hours |
| | mm | mm | mm | mm | mm | mm |
| 200-242B | 0.403 | 0.358 | 0.455 | 0.452 | 0.407 | 0.378 |
| | 0.403 | 0.353 | 0.458 | 0.455 | 0.408 | 0.381 |
| | 0.404 | 0.354 | 0.458 | 0.459 | 0.409 | 0.379 |
| | 0.419 | 0.36 | 0.375 | 0.398 | 0.39 | 0.391 |
| | 0.417 | 0.361 | 0.374 | 0.403 | 0.396 | 0.395 |
| | 0.421 | 0.366 | 0.38 | 0.41 | 0.398 | 0.399 |
| Mean ± SD | 0.411 ±.009 | 0.359 ±.005 | 0.417 ±.044 | 0.430 ±.029 | 0.401 ±.008 | 0.387 ±.009 |
| Healon PK66220 | 0.411 | 0.455 | 0.416 | 0.487 | 0.401 | 0.41 |
| | 0.414 | 0.456 | 0.417 | 0.486 | 0.405 | 0.411 |
| | 0.415 | 0.46 | 0.421 | 0.485 | 0.406 | 0.411 |
| | 0.411 | 0.341 | 0.471 | 0.4808 | 0.421 | 0.385 |
| | 0.411 | 0.342 | 0.467 | 0.412 | 0.421 | 0.386 |
| | 0.407 | 0.343 | 0.472 | 0.413 | 0.422 | 0.388 |
| Mean ± SD | 0.412 ±.003 | 0.400 ±.063 | 0.444 ±.029 | 0.449 ±.041 | 0.413 ±.010 | 0.399 ±.013 |

Table 3

| McDonald Shadduck Evaluation | | | | | | |
|---|---|---|---|---|---|---|
| | Pre | 1 Hour | 3 Hours | 5 Hours | 24 Hours | 48 Hours |
| | Total Score | Total Score | Total Score | Total Score | Total Score | Total Score |
| 200-242B | 0 | 2 | 5.5 | 6 | 4.5 | 3 |
| | 0.5 | 3 | 3 | 4 | 2.5 | 1.5 |
| Mean ± SD | .25 ±.35 | 2.5 ±.71 | 4.25 ±1.76 | 5.0 ±1.41 | 3.5 ±1.41 | 2.25 ±1.06 |
| Healon PK66220 | 1.5 | 3.5 | 5.5 | 6 | 3.5 | 2.5 |
| | 0.5 | 2.5 | 3.5 | 5 | 1.5 | 1.5 |
| Mean ± SD | 1.0 ±.71 | 3.0 ±.71 | 4.5 ±1.41 | 5.5 ±.71 | 2.5 ±1.41 | 2.0 ±.71 |

In measurements of intraocular pressure, no statistically significant differences were found between the Healon control group and the test group at any of the time periods monitored. Evaluated mean intraocular pressures were noted at 48 hours for the test group as compared to the Healon control group at the same time period. Normal intraocular pressures were maintained at all other time periods throughout the 48 hour observation period for both the test group and the Healon control group.

Corneal thickness measurements after anterior chamber injection with the test group exhibited moderate increases in corneal swelling over the first 5 hours post injection. At 3 and 24 hours post injection the Healon control group exhibited statistically significant increases in corneal swelling as compared to the test group. Normal corneal thicknesses were observed for the Healon control group and the test at 48 hours post injection.

Ocular irritation and inflammation, graded by the McDonald-Shadduck score system, showed no statistically significant differences at any of the time periods evaluated between the test group and the Healon control group. Mean scores throughout the 48 hour observation period were considered negative for the test and control groups. Minimal inflammatory responses were demonstrated with respect to aqueous flare, and accumulation of inflammatory cells in the anterior chamber for all samples. Minimal injection of the tertiary and secondary iris vessels were noted for both the test sample and the Healon control samples. The test group exhibited 1+ lens fiber swelling at 3 and 5 hours post injection. The test sample also exhibited a 1+ fibrin response noted at 24 hours post injection. A 1+ lens fiber swelling was also observed in the Healon control group at 3 hours post injection.

Example 3

A study was conducted to evaluate and quantitate the inflammatory response of sample 200-242B following injection into the vitreal chamber of New Zealand white rabbits. Parameters studied were the quantitation of inflammatory cells in the vitreous, and ocular irritation and inflammation as graded by the modified McDonald-Shadduck and Erwin Hultsch scoring systems (see Erwin Hultsch, Ophthalmology, 87, 7 (July 1980)).

Animals were examined prior to and 48 hours after intravitreal injection of the test materials. At each time interval, treated and control eyes and vitreous were examined and scored for ocular reactions according to modified McDonald-Shadduck and Erwin Hultsch score systems. This study was performed to provide information on the inflammatory response to the injected material in a rabbit model.

Under a surgical microscope the conjunctival sac was flushed with sterile BSS and a 6-0 silk suture was placed beneath the superior extraocular muscle and clamped with a hemostat to retract the eye to reduce ocular movement. A four to five millimeter incision was made through the conjunctiva and Tennon's capsule about 3 to 5 mm from the limbus to expose the sciera in a region of the parsplana just lateral to the superior rectus muscle. A 8-0 coated vicryl purse string type suture was then placed in the sciera in a circular fashion and left untied. A 19 gauge needle on a three cc syringe was then passed through the sciera in the area enclosed by the purse-string suture into the center of the vitreous humor. A small volume of vitreous (0.5 ml) was withdrawn as atraumatically as possible. This volume of vitreous was then replaced with an equal volume (0.5 ml) of test material. A 25 gauge needle was then inserted through the same puncture hole, and the test material was instilled into the center of the vitreous body. The needle was then withdrawn and the wound closed by tightening the purse-string suture. The 6-0 suture was removed and the eye was returned to the normal position and antibiotic ointment was applied topically.

At 48 hours post injection, 0.4 cc of the vitreous was removed by tapping the vitreous with a 19 gauge needle 90° from the original injection site. Cell counts and ocular inflammatory response were determined to evaluate the inflammatory response to the test material. Control eyes were also evaluated for inflammatory responses.

Table 4

| Rabbit Vitreal Replacement Study | | | | | | |
|---|---|---|---|---|---|---|
| Rabbit No. | Treatment | Eye | Pre Cell Count mm³ | Post Cell Count mm³ | Clini -cal Score* 48 Hours | Positive Score |
| 614 | 200-242B | OS | 10,0,0,0 | 0,0,0,0 | 1 | No |
|  | Control | OD |  | 0,0,0,0 | 1 | No |
| 629 | 200-242B | OS | 0,0,0,10 | 30,0,30,10 | 2 | No |
|  | Control | OD |  | 0,20,10,0 | 2 | No |

*Clinical scores based on cells/mm³ only, does not include aqueous and vitreous scoring

**Table 5**

**Scoring the Inflammatory Response After Intravitreal Injection 48 Hour Evaluation**

| Rabbit No. | Treatment | Eye | Aqueous Flare | Cells | Vitreal Cells/mm³ | Vitreal Haze Ocular Fundus | Keratic Precipitate | Clinical Score | Positive Score |
|---|---|---|---|---|---|---|---|---|---|
| 614 | 200-242B | OS | 1 | 2 | 1 | 1 | 1 | 1.2 | No |
|  | Control | OD | 1 | 1 | 1 | 1 | 1 | 1.0 | No |
| 629 | 200-242B | OS | 1 | 4 | 1 | 1 | 1 | 1.6 | No |
|  | Control | OD | 1 | 1 | 1 | 1 | 1 | 1.0 | No |

Animals were examined prior to and 48 hours after intravitreal injection of the test materials. At each time interval, treated and control eyes and vitreous were examined and scored for ocular reactions according to modified McDonald-Shadduck and Erwin Hultsch score systems. Clinical findings in combination with vitreous humor cell counts allows a reliable evaluation of the inflammatory potential of the test material. The test material is considered non-inflammatory if the overall mean vitreal cell count is less than

50 cell/mm$^3$ and the overall clinical response is less than 2.0 (see Table 6).

Mean vitreal cell counts 48 hours post injection ware $9 \pm 14$ cells/mm$^3$ for the test group and $4 \pm 8$ cells/mm$^3$ for the control group. Both of these mean vitreal cell counts are considered non-inflammatory by the Hultsch score system. Aqueous flare, vitreal cells, vitreal haze, ocular fundus and keratic precipitates scores were rated as non-inflammatory for the treatment and control eyes. The treatment group exhibited a moderate to severe anterior chamber cell reaction. Mean clinical scores for both the treatment (1.4) and the control (1.0) eyes were considered non-inflammatory.

Test sample 20-242B was considered non-inflammatory exhibiting a mean vitreal cell count less than 50 cells/mm$^3$ and a clinical score less than 2.0

Table 6

Grading of the Inflammatory Response After Intravitreal Injection

| Clinical Grade | Aqueous Flare | Cells | Vitreous Cells/mm³ | Vitreous Haze Ocular Fundus | Keratic Precipitates |
|---|---|---|---|---|---|
| 1 | Barely Detectable | None | 0 | No Haze Fundus Clear | None |
| 2 | Slight | Very Few | <50 | No Haze Fundus Clear | None |
| 3 | Moderate | Moderate Number | 51-200 | Definite Haze Fundus Clear | Just Detectable |
| 4 | Moderate-Severe | Many | 201-600 | Marked Haze Fundus Details Visible | Small |
| 5 | Severe | Masses | 601-1200 | Severe Haze Red Fundus Reflex No Fundus Details Visible | Medium |
| 6 | Severe | Masses | >1200 | Severe Haze Gray Fundus Reflex | Large |

Example 4

A study was undertaken to evaluate ocular inflammation produced by sample 300-37B against a control of Healon® lot No. P166216) following the procedure described in Example 2.

11

Table 7

| Intraocular Pressure Evaluation | | | | | | |
|---|---|---|---|---|---|---|
| | Pre | 1 Hour | 3 Hours | 5 Hours | 24 Hours | 48 Hours |
| | mm Hg | mm Hg | mm Hg | mm Hg | mm Hg | mm Hg |
| 300-37B | 12.2<br>12.0<br>9.6 | 14.2<br>12.5<br>10.8 | 31.4<br>15.8<br>5.0 | 33.6<br>14.2<br>13.2 | 21.6<br>18.4<br>6.6 | 18.7<br>17.4<br>12.6 |
| Mean ± SD | 11.3 ±1.4 | 12.5 ±1.7 | 17.4 ±13.2 | 20.3 ±11.5 | 15.5 ±7.9 | 16.2 ±3.7 |
| Healon P166216 | 11.6<br>8.8<br>14.2 | 5.3<br>16.4<br>5.4 | 5.7<br>16.3<br>5.6 | 6.6<br>32.2<br>5.5 | 7.7<br>11.7<br>15.1 | 11.2<br>10.8<br>12.5 |
| Mean ± SD | 11.5 ±2.7 | 9.0 ±6.3 | 9.2 ±6.1 | 14.7 ±15.1 | 11.5 ±3.7 | 11.5 ±0.9 |

Table 8

| Corneal Thickness Evaluation | | | | | | |
|---|---|---|---|---|---|---|
| | Pre | 1 Hour | 3 Hours | 6 Hours | 24 Hours | 48 Hours |
| | mm | mm | mm | mm | mm | mm |
| 300-37B | 0.396<br>0.404<br>0.411<br>0.394<br>0.398<br>0.403<br>0.396<br>0.398<br>0.405 | 0.413<br>0.414<br>0.417<br>0.406<br>0.419<br>0.423<br>0.435<br>0.444<br>0.437 | 0.419<br>0.413<br>0.421<br>0.384<br>0.386<br>0.389<br>0.425<br>0.430<br>0.426 | 0.413<br>0.413<br>0.415<br>0.380<br>0.384<br>0.394<br>0.378<br>0.382<br>0.389 | 0.404<br>0.406<br>0.406<br>0.398<br>0.401<br>0.402<br>0.409<br>0.407<br>0.408 | 0.356<br>0.359<br>0.358<br>0.364<br>0.368<br>0.367<br>0.391<br>0.397<br>0.401 |
| Mean ± SD | 0.401 ±.006 | 0.423 ±.013 | 0.410 ±.019 | 0.394 ±.015 | 0.405 ±.004 | 0.373 ±.018 |
| Healon P166216 | 0.387<br>0.399<br>0.404<br>0.358<br>0.364<br>0.365<br>0.362<br>0.369<br>0.372 | 0.443<br>0.443<br>0.446<br>0.436<br>0.449<br>0.437<br>0.472<br>0.476<br>0.477 | 0.428<br>0.431<br>0.427<br>0.430<br>0.442<br>0.430<br>0.411<br>0.413<br>0.415 | 0.454<br>0.459<br>0.467<br>0.394<br>0.397<br>0.400<br>0.418<br>0.421<br>0.426 | 0.420<br>0.417<br>0.418<br>0.424<br>0.425<br>0.425<br>0.394<br>0.397<br>0.398 | 0.398<br>0.402<br>0.405<br>0.335<br>0.340<br>0.340<br>0.379<br>0.380<br>0.384 |
| Mean ± SD | 0.376 ±.017 | 0.453 ±.017 | 0.425 ±.010 | 0.426 ±.028 | 0.413 ±.013 | 0.402 ±.019 |

Table 9

| McDonald Shadduck Evaluation | | | | | | |
|---|---|---|---|---|---|---|
| | Pre | 1 Hour | 3 Hours | 5 Hours | 24 Hours | 48 Hours |
| | Total Score | Total Score | Total Score | Total Score | Total Score | Total Score |
| 300-37B | 1.5<br>2.0<br>1.5 | 3.0<br>3.0<br>3.0 | 2.5<br>4.0<br>5.5 | 2.5<br>4.0<br>3.5 | 0.5<br>1.5<br>1.5 | 1.0<br>1.0<br>1.0 |
| Mean ± SD | 1.66 ±.29 | 3.0 ±.0 | 4.0 ±1.5 | 3.33 ±.76 | 1.16 ±.57 | 1.0 ±0 |
| Healon P166216 | 1.5<br>2.5<br>1.0 | 3.0<br>3.5<br>3.0 | 3.0<br>3.5<br>4.0 | 6.0<br>4.0<br>4.5 | 4.5<br>3.5<br>1.0 | 2.5<br>2.0<br>0.5 |
| Mean ± SD | 1.66 ±.76 | 3.16 ±.29 | 3.5 ±.50 | 4.83 ±1.04 | 3.0 ±1.80 | 1.66 ±1.04 |

In measurements of intraocular pressure, no statistically significant differences were found between the Healon control group and the test group at any of the time periods monitored. Increased intraocular pressures were observed in the treatment group at 3, 6, 24, and 48 hours post injection. The greatest increase in intraocular pressure was observed at 6 hours post injection for the treatment group (20.3 ± 11.5 mm Hg) as compared to the Healon control group (14.7 ± 15.1 mm Hg).

Normal corneal thickness measurements after anterior chamber injection with the test group throughout the 48 hour observation period. The Healon control group exhibited statistically significant increases in corneal thickness at both 1 and 6 hours post injection as compared to the test group. Corneal thickness measurements at 1 and 6 hours for the treatment group and the Healon control group ware .423 ± .013 mm and .453 ± .017 mm (1 hour); .394 ± .015 mm and .426 ± .028 mm (6 hours), respectively. Normal corneal thicknesses were observed for both the test group and the Healon control group at 24 and 48 hours post injection.

Ocular irritation and inflammation, graded by the McDonald-Shadduck score system, showed no statistically significant differences at any of the time periods evaluated between the test group and the Healon control group. Mean scores throughout the 48 hour observation period were considered negative for the test and control groups. Minimal injection of the tertiary and secondary iris vessels were noted for both the tent sample and the Healon control samples. Minimal injection of the conjunctive vessels was also observed during the first 6 hours post injection for both the test group and the Healon control. A transient suture line (2.5-1) was observed during the first hour post injection for the test group. Additional lens changes for the test group included (2-2.5) formation of microprecipitates immediately post injection. Transient lens fiber swelling (.5-1) was also observed in the test group during the first 6 hours post injection. The Healon control group also exhibited (.5-1) transient lens fiber swelling during the first 3 hours post injection. Minimal inflammatory responses were demonstrated with respect to aqueous flare, and accumulation of inflammatory cells in the anterior chamber for all samples.

Example 5

A study was undertaken to evaluate ocular inflammation produced by sample 200-302 against a control of Healon® lot No. PK66220) following the procedure described in Example 2.

Table 10

| Intraocular Pressure Evaluation | | | | | | |
|---|---|---|---|---|---|---|
| | Pre | 1 Hour | 3 Hours | 5 Hours | 24 Hours | 48 Hours |
| | mm Hg | mm Hg | mm Hg | mm Hg | mm Hg | mm Hg |
| 200-302 | 12.8<br>11.8<br>9.5 | 11.32<br>12.1<br>5.9 | 13.3<br>12.3<br>12.7 | 11.1<br>22<br>20.9 | 7.5<br>10.8<br>6 | 6.3<br>10.3<br>12.8 |
| Mean ± SD | 11.4 ±1.7 | 9.8 ±3.4 | 12.8 ±.5 | 18.0 ±6 | 8.1 ±2.5 | 9.8 ±3.3 |
| Healon P166216 | 10.7<br>12.1<br>6.3 | 12.6<br>6.1<br>12.3 | 21.9<br>13.3<br>25.1 | 22.4<br>25.1<br>28.7 | 10.1<br>12.4<br>15.1 | 9.2<br>11.1<br>7.4 |
| Mean ± SD | 9.7 ±3.0 | 10.4 ±3.7 | 20.1 ±6.1 | 25.4 ±3.2 | 12.5 ±2.5 | 9.2 ±1.9 |

Table 11

| Corneal Thickness Evaluation | | | | | | |
|---|---|---|---|---|---|---|
| | Pre | 1 Hour | 3 Hours | 6 Hours | 24 Hours | 48 Hours |
| | mm | mm | mm | mm | mm | mm |
| 200-302 | 0.381<br>0.386<br>0.378<br>0.423<br>0.425<br>0.425<br>0.400<br>0.402<br>0.402 | 0.404<br>0.405<br>0.405<br>0.391<br>0.397<br>0.398<br>0.407<br>0.403<br>0.409 | 0.416<br>0.417<br>0.419<br>0.409<br>0.410<br>0.411<br>0.416<br>0.417<br>0.420 | 0.398<br>0.407<br>0.406<br>0.431<br>0.434<br>0.437<br>0.411<br>0.407<br>0.404 | 0.433<br>0.435<br>0.438<br>0.441<br>0.443<br>0.445<br>0.398<br>0.412<br>0.412 | 0.399<br>0.402<br>0.456<br>0.411<br>0.415<br>0.413<br>0.396<br>0.400<br>0.401 |
| Mean ± SD | 0.402 ±.019 | 0.402 ±.006 | 0.415 ±.004 | 0.415 ±.015 | 0.429 ±.017 | 0.405 ±.007 |
| Healon PK66220 | 0.397<br>0.403<br>0.403<br>0.406<br>0.406<br>0.406<br>0.398<br>0.370<br>0.374 | 0.438<br>0.444<br>0.445<br>0.360<br>0.360<br>0.361<br>0.433<br>0.431<br>0.428 | 0.433<br>0.437<br>0.435<br>0.402<br>0.406<br>0.405<br>0.419<br>0.421<br>0.429 | 0.447<br>0.449<br>0.450<br>0.419<br>0.418<br>0.407<br>0.413<br>0.416<br>0.416 | 0.405<br>0.409<br>0.409<br>0.437<br>0.439<br>0.440<br>0.427<br>0.432<br>0.432 | 0.400<br>0.399<br>0.400<br>0.419<br>0.423<br>0.426<br>0.390<br>0.397<br>0.388 |
| Mean ± SD | 0.393 ±.017 | 0.411 ±.038 | 0.421 ±.014 | 0.426 ±.017 | 0.426 ±.014 | 0.405 ±.014 |

Table 12

| McDonald Shadduck Evaluation | | | | | | |
|---|---|---|---|---|---|---|
| | Pre | 1 Hour | 3 Hours | 5 Hours | 24 Hours | 48 Hours |
| | Total Score | Total Score | Total Score | Total Score | Total Score | Total Score |
| 200-302 | 0.5<br>1<br>2 | 1<br>1.5<br>1.5 | 1.5<br>2.5<br>3.5 | 2<br>2.5<br>5 | 3<br>2.5<br>5 | 0<br>1.5<br>2.5 |
| Mean ± SD | 1. 6 ±.8 | 1. 3 ±.3 | 2.5 ±1.0 | 3.2 ±1.6 | 3.5 ±1.3 | 1.3 ±1.3 |
| Healon PK66220 | 0.5<br>1<br>2 | 1<br>1.5<br>1.5 | 2<br>2.5<br>4 | 1.5<br>2<br>4 | 2.5<br>3<br>5 | 0<br>2.5<br>3 |
| Mean ± SD | 1.2 ±.8 | 1.3 ±.3 | 2.8 ±1.0 | 2.5 ±1.3 | 3.5 ±1.3 | 1.8 ±1.6 |

In measurements of intraocular pressure, no statistically significant differences were found between the Healon control group and the test group at any of the time periods monitored due to the small sampling size. Elevated mean intraocular pressures were noted at 3 and 5 hours for the Healon control group as compared to the test group at the same time periods. Normal intraocular pressures were maintained at all other time periods throughout the 48 hour observation period for both the test group and the Healon control group.

Corneal thickness measurements after anterior chamber injection with the test group exhibited minimal increases in corneal swelling over the first 5 hours post injection. No statistically significant differences in corneal thicknesses were found between the Healon control group and the test group at any of the time periods monitored. Normal corneal thicknesses were maintained throughout the 48 hour observation period for both the test and control groups.

Ocular irritation and inflammation, graded by the McDonald-Shadduck score system, showed no statistically significant differences at any of the time periods evaluated between the test group and the Healon control group. Mean scores throughout the 48 hour observation period were considered negative for the test and control groups. Minimal inflammatory responses were demonstrated with respect to aqueous flare, and accumulation of inflammatory cells in the anterior chamber for all samples. Minimal injection of the tertiary and secondary iris vessels were noted for both the test sample and the Healon control samples.

Example 6

A study was conducted to evaluate and quantitate the inflammatory response of sample 200-242B following the procedure of Example 3.

Table 13

| Rabbit Vitreal Replacement Study | | | | | | |
|---|---|---|---|---|---|---|
| Rabbit No. | Treatment | Eye | Pre Cell Count mm$^3$ | Post Cell Count mm$^3$ | Clinical Score* 48 Hours | Positive Score |
| 1007 | 200-302B<br>Control | OS<br>OD | 0,0,0,0 | 0,30,0,30<br>0,0,0,0 | 2<br>1 | No<br>No |
| 1008 | 200-302B<br>Control | OS<br>OD | 0,10,10,0 | 0,10,20,0<br>10,0,0,0 | 2<br>2 | No<br>No |

*Clinical scores based on cells/mm$^3$ only, does not include aqueous and vitreous scoring

**Table 14**

**Scoring the Inflammatory Response After Intravitreal Injection**
**48 Hour Evaluation**

| Rabbit No. | Treatment | Eye | Aqueous Flare | Cells | Vitreal Cells/mm³ | Vitreal Haze Ocular Fundus | Keratic Precipitate | Clinical Score | Positive Score |
|---|---|---|---|---|---|---|---|---|---|
| 1007 | 200-302 | OS | 1 | 1 | 2 | 1 | 1 | 1.2 | No |
|  | Control | OD | 1 | 1 | 1 | 1 | 1 | 1.0 | No |
| 1008 | 200-302 | OS | 1 | 1 | 2 | 1 | 1 | 1.2 | No |
|  | Control | OD | 1 | 1 | 1 | 1 | 1 | 1.0 | No |

Example 7

A study was undertaken to evaluate ocular inflammation produced by sample 300-39A against a control of Healon® lot No. P166216) following the procedure described in Example 2.

Table 15

| Intraocular Pressure Evaluation | | | | | | |
|---|---|---|---|---|---|---|
| | Pre | 1 Hour | 3 Hours | 5 Hours | 24 Hours | 48 Hours |
| | mm Hg | mm Hg | mm Hg | mm Hg | mm Hg | mm Hg |
| 300-39A | 11.3<br>13.5<br>8.6 | 16.5<br>5.1<br>5.0 | 12.5<br>5.8<br>6.1 | 7.7<br>5.6<br>9.7 | 20.4<br>6.8<br>16.7 | 17.2<br>12.5<br>16.2 |
| Mean ± SD | 11.1 ±2.5 | 8.9 ±6.6 | 8.1 ±3.8 | 7.7 ±2.1 | 14.6 ±7.0 | 15.3 ±2.5 |
| Healon P166216 | 7.4<br>12.3<br>12.8 | 6.0<br>7.9<br>5.4 | 5.0<br>24.1<br>7.5 | 16.0<br>23.7<br>9.7 | 5.7<br>15.7<br>12.9 | 10.2<br>13.1<br>12.4 |
| Mean ± SD | 10.8 ±2.9 | 6.4 ±1.3 | 12.2 ±10.3 | 16.4 ±7.0 | 11.4 ±5.2 | 11.9 ±1.5 |

Table 16

| Corneal Thickness Evaluation | | | | | | |
|---|---|---|---|---|---|---|
| | Pre | 1 Hour | 3 Hours | 6 Hours | 24 Hours | 48 Hours |
| | mm | mm | mm | mm | mm | mm |
| 300-39A | 0.403<br>0.403<br>0.407<br>0.450<br>0.454<br>0.455<br>0.417<br>0.415<br>0.420 | 0.401<br>0.404<br>0.405<br>0.437<br>0.447<br>0.440<br>0.467<br>0.465<br>0.463 | 0.405<br>0.409<br>0.414<br>0.421<br>0.417<br>0.423<br>0.405<br>0.406<br>0.409 | 0.416<br>0.419<br>0.418<br>0.416<br>0.416<br>0.423<br>0.386<br>0.389<br>0.389 | 0.408<br>0.406<br>0.406<br>0.445<br>0.449<br>0.449<br>0.444<br>0.446<br>0.447 | 0.425<br>0.425<br>0.424<br>0.418<br>0.422<br>0.422<br>0.398<br>0.402<br>0.403 |
| Mean ± SD | 0.425 ±.022 | 0.437 ±.027 | 0.412 ±.007 | 0.408 ±.015 | 0.433 ±.020 | 0.415 ±.011 |
| Healon P166216 | 0.407<br>0.407<br>0.405<br>0.439<br>0.439<br>0.449<br>0.414<br>0.414<br>0.416 | 0.450<br>0.457<br>0.455<br>0.490<br>0.484<br>0.484<br>0.450<br>0.450<br>0.449 | 0.425<br>0.427<br>0.427<br>0.442<br>0.443<br>0.452<br>0.425<br>0.427<br>0.431 | 0.421<br>0.417<br>0.419<br>0.441<br>0.440<br>0.442<br>0.416<br>0.419<br>0.420 | 0.388<br>0.394<br>0.399<br>0.455<br>0.467<br>0.462<br>0.426<br>0.432<br>0.430 | 0.412<br>0.413<br>0.413<br>0.433<br>0.434<br>0.438<br>0.411<br>0.412<br>0.414 |
| Mean ± SD | 0.421 ±.017 | 0.463 ±.017 | 0.433 ±.010 | 0.426 ±.011 | 0.428 ±.030 | 0.420 ±.011 |

Table 17

| McDonald Shadduck Evaluation | | | | | | |
|---|---|---|---|---|---|---|
| | Pre | 1 Hour | 3 Hours | 5 Hours | 24 Hours | 48 Hours |
| | Total Score | Total Score | Total Score | Total Score | Total Score | Total Score |
| 300-39A | 2.0<br>0.0<br>2.0 | 4.0<br>3.5<br>4.0 | 5.5<br>4.5<br>5.5 | 6.0<br>4.5<br>6.5 | 1.5<br>2.5<br>1.5 | 1.5<br>0.0<br>1.0 |
| Mean ± SD | 1.33 ±1.15 | 3.83 ±.28 | 5.16 ±1.04 | 5.66 ±1.04 | 1.83 ±.57 | .83 ±.76 |
| Healon P166216 | 2.5<br>0.0<br>1.0 | 4.5<br>2.0<br>3.5 | 6.0<br>4.0<br>4.0 | 7.0<br>5.0<br>6.0 | 1.5<br>1.5<br>1.5 | 1.5<br>1.0<br>1.5 |
| Mean ± SD | 1.16 ±1.25 | 3.33 ±1.25 | 4.66 ±1.15 | 6.0 ±1.0 | 1.50 ±0 | 1.33 ±.28 |

Corneal thickness measurements after anterior chamber injection with the test group exhibited corneal thinning during the first 6 hours post injection. The Healon control exhibited statistically significant increases in corneal thickness at both 3 and 6 hours post injection as compared to the test group. Corneal thickness measurements at 3 and 6 hours for the treatment group and the Healon control group were .412 ± .007 mm and .433 ± .010 mm (3 hours); .408 ± .015 and .426 ± .011 mm (6 hours), respectively. Normal corneal thicknesses were observed for both the test group and the Healon control group at 24 and 48 hours post injection.

Ocular irritation and inflammation, graded by the McDonald-Shadduck score system, showed no statistically significant differences at any of the time periods evaluated between the test group and the Healon control group. Mean scores throughout the 48 hour observation period were considered negative for the test and control groups. Moderate injection of the tertiary and secondary iris vessels were noted for both the test sample and the Healon control samples. Moderate injection of the conjunctival vessels was also observed during the first 6 hours post injection for both the test group and the Healon control. A transient suture line (1-1.5) was observed during the first 3 hours post injection for the test group.

**Claims**

1.  An aqueous viscoelastic solution of N,O-carboxymethyl chitosan or physiologically acceptable salts thereof, or cross-linked N,O-carboxymethyl chitosan.

2.  An aqueous viscoelastic N,O-carboxymethyl chitosan solution as defined in Claim 1, comprising from about 0.5 to about 3 % by weight of said N,O-carboxymethyl chitosan or physiologically acceptable salts thereof or cross-liked N,O-carboxymethyl chitosan, the balance being a physiologically acceptable vehicle.

3.  An aqueous viscoelastic N,O-carboxymethyl chitosan solution as defined in Claim 1, wherein said salt is the acetate, lactate, citrate, or pyroglutamate.

4.  An aqueous viscoelastic N,O-carboxymethyl chitosan solution as defined in Claim 1, having a pH of from about 7.2 to about 7.4 and an osmolality of from about 300 to about 340.

5.  An aqueous viscoelastic N,O-carboxymethyl chitosan solution as defined in Claim 1, which is sterile and non-pyrogenic.

6.  An aqueous viscoelastic N,O-carboxymethyl chitosan solution as defined in Claim 5, comprising from about 0.5 to about 3 % by weight of said N,O-carboxymethyl chitosan or physiologically acceptable salts thereof or cross-linked N,O-carboxymethyl chitosan, the balance being a physiologically acceptable vehicle.

7.  An aqueous viscoelastic N,O-carboxymethyl chitosan solution as defined in Claim 5, wherein said salt is the acetate, lactate, citrate, or pyroglutamate.

8.  An aqueous viscoelastic N,O-carboxymethyl chitosan solution as defined in Claim 5, having a pH of from about 7.2 to about 7.4 and an osmolality of from about 300 to about 340.

9.  A method of replacing at least a portion of the aqueous or vitreous humor in a human or animal eye comprising introducing into the aqueous or vitreous chamber of said human or animal eye an effective amount of a viscoelastic N,O-carboxymethyl chitosan solution as defined in Claim 1.

10. A method of inserting an intraocular lens into the anterior or posterior chamber of a human or animal eye comprising introducing into said anterior or posterior chamber an amount of a viscoelastic N,O-carboxymethyl chitosan solution as defined in Claim 1, sufficient to reduce the trauma which normally results from inserting an intraocular lens into said chamber without the use of a viscoelastic ophthalmic aid.

European Patent Office

## PARTIAL EUROPEAN SEARCH REPORT

which under Rule 45 of the European Patent Convention
shall be considered, for the purposes of subsequent
proceedings, as the European search report

Application Number

EP 94 30 0663

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | EP-A-0 426 368 (PFIZER) <br> * claims 1-3,13; examples 4,6,8 * <br> ----- | 1-3,5-7 | A61L25/00 <br> A61L31/00 <br> C08L5/08 <br> C08B37/00 |
| | | | **TECHNICAL FIELDS SEARCHED (Int.Cl.6)** <br> A61L <br> C08L <br> C08B |

## INCOMPLETE SEARCH

The Search Division considers that the present European patent application does not comply with
the provisions of the European Patent Convention to such an extent that it is not possible to carry
out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 28 June 1994 | Peltre, C |

EPO FORM 1503 03.82 (P04C07)

EP 94 30 0663

-C-

Remark: Although claims 9,10
are directed to a method of
treatment of the human/animal
body (Art. 52(4) EPC) the search
has been carried out and based on
the alleged effects of the
composition